# EUROPÄISCHE PATENTSCHRIFT

(11) **EP 0 697 107 B1**
(45) Veröffentlichungstag und Bekanntmachung des Hinweises auf die Patenterteilung: **07.01.1998**
(21) Anmeldenummer: 94916160.8
(22) Anmeldetag: 27.04.1994
(51) Int. Cl.: G01N 21/66, G01N 33/52, G01N 33/58

(54) **ELEKTROCHEMILUMINESZENZVERFAHREN**
ELECTROCHEMILUMINESCENT PROCESS
PROCEDE ELECTROCHIMIOLUMINESCENT

(30) Priorität: 03.05.1993 DE 4314547; 25.09.1993 DE 4332697; 20.01.1994 DE 4401577
(43) Veröffentlichungstag der Anmeldung: 21.02.1996
(73) Patentinhaber: BOEHRINGER MANNHEIM GMBH, 68298 Mannheim (DE)
(72) Erfinder: GIESEN, Ursula, D-82362 Weilheim (DE); HOYLE, Nicholas, D-82327 Tutzing (DE); KLEMT, Volker, D-82362 Weilheim (DE); MÜLLER, Günter, D-82380 Peissenberg (DE); NEUMANN, Ulrich, D-82362 Weilheim (DE)
(86) Internationale Anmeldenummer: EP9401329
(87) Internationale Veröffentlichungsnummer: WO9425854

(56) Entgegenhaltungen:
- WO-A-89/10551
- WO-A-90/05296
- WO-A-90/05411
- J. ELECTROCHEM. SOC., Bd.137, Nr.10, Oktober 1990 Seiten 3127 - 3131 J. K. LELAND ET AL. 'ELECTROGENERATED CHEMILUMINESCENCE: AN OXIDATIVE-REDUCTION TYPE ECL REACTION SEQUENCE USING TRIPROPYL AMINE.'
- INORGANIC CHEMISTRY, Bd.29, Nr.19, 19. September 1990 Seiten 3711 - 3717 R. D. MUSSELL ET AL. 'PARTITIONING OF THE ELECTROCHEMICAL EXCITATION ENERGY IN THE ELECTROGENERATED CHEMILUMINESCENCE OF HEXANUCLEAR MOLYBDENUM AND TUNGSTEN CLUSTERS.'

## Beschreibung

Gegenstand der Erfindung sind Verfahren zur Messung elektrochemilumineszenter Phänomene, Verfahren zum Nachweis eines Analyten mittels solcher Verfahren, Reagenzlösungen, die in diesen Verfahren eingesetzt werden können und ein für die Durchführung des Verfahrens besonders geeigneter Apparat.

Verfahren zur Messung elektrochemilumineszenter Phänomene sind seit einigen Jahren bekannt. Bei solchen Verfahren wird die Fähigkeit von speziellen Metallkomplexen ausgenutzt, durch Oxidation in einen Zustand zu gelangen, von dem aus sie unter Abgabe von elektromagnetischer Strahlung in einen Grundzustand zurückfallen. Solche Verfahren und dafür geeignete Metallkomplexe sind beispielsweise in der WO 86/02734 beschrieben.

Diese Technologie wurde immer weiter verfeinert. In der WO 90/05296 wird der Testzusammensetzung ein Amin zugesetzt, bevorzugt Tripropylamin, das in oxidierter Form ein starkes Reduktionsmittel darstellt. Die elektrochemische Reaktion findet in einem Elektrolyten statt, in dem der Elektrochemilumineszenz (ECL)-Rest, d. h. der zur Abgabe von elektromagnetischer Strahlung befähigte Metallkomplex, und das Amin oxidiert werden können. Als geeigneter Elektrolyt in wässriger Lösung wird Phosphatpuffer bei einem pH von 6 - 9, bevorzugt 7 - 7,5, beschrieben. In der WO 90/05302 wird zu dieser Testzusammensetzung zur Erhöhung der elektromagnetischen Strahlung des Detergenz Triton X-100 oder Triton N-401 zugesetzt. In der WO 90/05411 wird eine Verbesserung des Apparates zur Messung von ECL beschrieben.

Ferner gelang es, die Technologie zum Nachweis von Analyten einzusetzen, indem Elektrochemilumineszenzmarkierungen an Analyten, Analytanaloge oder analytspezifische Substanzen gekoppelt wurden. Die Elektrochemilumineszenz wurde zur Bestimmung der Menge des anwesenden Analyten verwendet. Insbesondere wurden Immunoassays beschrieben, bei denen die üblichen Markierungen durch elektrochemilumineszente Markierungen ersetzt sind.

In Inorganic Chemistry, Bd. 29, Nr. 19, Sept. 1990, S. 3711-3717 wird die Messung von Elektrochemilumineszenz in organischer Lösung beschreiben. Die Messungen werden bei etwa 23° C ausgeführt, wobei nicht darauf hingewiesen wird, daß die Meßtemperatur einen Einfluß auf die Empfindlichkeit der Messung besitzt. Außerdem sind die Meßbedingungen in organischer Lösung nicht mit den Bedingungen in wässriger Lösung gleichzusetzen.

Weitere Verbesserungen und Anwendungen der Technologie sind in WO 87/06706, WO 89/04392, WO 89/10552, WO 89/10551, WO 90/05301 und WO 90/11511 beschrieben. Die Offenbarungen in den genannten Publikationen werden im folgenden vorausgesetzt.

Aufgabe der vorliegenden Erfindung war es, die vorbekannten Verfahren zu verbessern, insbesondere im Hinblick auf die Empfindlichkeit der Analytnachweise, die mit der Elektrochemilumineszenztechnologie möglich sind.

Gegenstand der Erfindung ist ein Verfahren zur Messung elektrochemilumineszenter Phänomene in einer wässrigen Lösung oder an einer an die wässrige Lösung angrenzenden festen Phase, wobei die Lösung ein oxidierbares Amin und eine zur Elektrochemilumineszenz fähige Komponente enthält, Anlegen einer Spannung zur Anregung der Elektrochemilumineszenz und Messung des ausgesendeten Lichtes als Maß für die Anwesenheit der zur Elektrochemilumineszenzfähigen Komponente, wobei die Messung der Elektrochemilumineszenz bei einer Temperatur der Lösung oder/und der festen Phase durchgeführt wird, die über dem Gefrierpunkt der Lösung liegt, aber niedriger als 25°C ist.

Ferner ist Gegenstand der Erfindung ein Verfahren zur Messung elektrochemilumineszenter Phänomene durch Anregung von Elektrochemilumineszenz durch Anlegen einer Spannung an eine Arbeitselektrode, wobei vor Anregen der Elektrochemilumineszenz an die Arbeitselektrode ein Potential zwischen + 400 und - 400 mV verglichen mit einer Ag/AgCl-Referenzelektrode angelegt wird.

Ferner ist Gegenstand der Erfindung ein Verfahren zur Messung elektrochemilumineszenter Phänomene durch Anregung von Elektrochemilumineszenz durch Anlegen einer Spannung an eine Arbeitselektrode, wobei die Elektrochemilumineszenz durch Anlegen einer Spannung zwischen dem Redoxpotential des elektrolumineszenten Systems und + 800 mV darüber angeregt wird.

Ferner ist Gegenstand der Erfindung ein Verfahren zur Messung elektrochemischer Phänomene in einer Lösung oder an einer an eine Lösung angrenzende feste Phase, wobei die Lösung ein Detergenz ausgewählt aus der Gruppe Thesit, C14-E09, Genapol, C8-E09, Plantaren oder Octylglucosid oder Gemische hiervon enthält.

Ferner ist Gegenstand der Erfindung ein Verfahren zum Nachweis eines Analyten mit Hilfe dieser Verfahren, geeignete Reagenzien und ein Apparat zur Durchführung des erstgenannten Verfahrens.

Bei dem Erfindungsgegenstand handelt es sich um eine Lehre, die auf dem oben genannten Stand der Technik aufbaut. Die allgemeinen Grundlagen elektrochemilumineszenter Verfahren sind in diesem Stand der Technik ausführlich beschrieben. Geräte zur Durchführung von Messungen der Elektrochemilumineszenz enthalten eine Meßeinheit mit einem Behälter für eine Reagenzlösung, mindestens zwei Elektroden (eine Arbeits- und eine Gegenelektrode), die während der Messung mit der Reagenzlösung in Kontakt stehen und einen Detektor zur Messung des durch die Elektrochemilumineszenz erzeugten Lichtes. Generell wird bei diesen Verfahren zunächst an die Lösung eine Ausgangsspannung (Präpolarisation) angelegt. Anschließend wird diese Spannung über das Redoxpotential einer in der Lösung enthaltenen Substanz, z. B. einem Amin, erhöht. Uber die dadurch oxidierte Substanz wird ein zur Chemilumineszenz fähiges Material, z. B. bestimmte Ruthenium-Komplexe zur Aussendung von Licht angeregt. Das innerhalb einer bestimmten Zeit vom Detektor aufgefangene Licht ist ein Maß für die Anwesenheit der Menge des elektrochemilumineszenten Materials. Sofern es sich bei dem elektrochemilumineszenten Material um eine Markierung für einen Analyten, einen Analytanalogen oder eine analytspezifische Substanz, z. B. in einem Immunoassay, handelt, ist das empfangene Licht ein Maß für die Anwesenheit des Analyten.

Während die bisherigen elektrochemilumineszenten Verfahren bei Temperaturen größer oder gleich 28 °C arbeiteten, wurde nun gefunden, daß bei Verwendung einer Rampenspannung mit sinkenden Temperaturen zwar die in der Zeiteinheit gemessene Signalstärke abnimmt, jedoch die Empfindlichkeit überraschenderweise erheblich zunimmt. Diese Verbesserung scheint auf eine erhebliche Verringerung der unspezifischen Bindung von ECL-markierten Konjugaten z. B. an Elektroden zurückzuführen zu sein. Bei einer Temperatur von 10 °C ist der Beitrag der unspezifischen Bindung nicht mehr von dem von der Reagenzlösung herruhrenden Anteils des Hintergrunds zu unterscheiden. Es hat sich herausgestellt, daß Temperaturen zwischen 5 - 20 °C bevorzugt und von 9 - 11 °C besonders bevorzugt sind. Die Erreichung solcher Temperaturen ist entweder möglich durch Kühlung der Reagenzlösung, bevor sie mit der Meßeinheit in Kontakt gebracht wird, oder/und durch Kühlung in der Meßeinheit selbst.

Ferner hat es sich als vorteilhaft erwiesen, die an die Arbeitselektrode angelegte Endspannung (verglichen mit Ag/AgCl) auf einen Maximalwert zwischen dem Redox-Potential der oxidierbaren Substanz und 2,0 Volt zu begrenzen. Besonders bevorzugt ist eine Spannung zwischen 1,2 - 1,7 Volt. Besonders bevorzugt sind 1,6 Volt. Diese Werte gelten für die Verwendung eines Platin-Elektrodenpaars.

Eine weitere Verbesserung der Empfindlichkeit kann erreicht werden, indem an die Meßeinheit eine Spannung angelegt wird, deren Verlauf über die Zeit rechteckig ist. Dies bedeutet, daß ausgehend von der Ausgangsspannung ein unmittelbarer Anstieg der Spannung (innerhalb maximal 0,4 Sekunden) auf die oben genannte Endspannung vorgenommen wird. Für die oben genannte Anregungszeit wird diese Spannung im wesentlichen konstant gehalten. Nach dieser Zeit wird die Spannung wieder unmittelbar auf eine Spannung unter dem Redoxpotential des Systems zurückgeführt. Durch diese Maßnahme ergibt sich außerdem eine Verbreiterung des dynamischen Meßbereiches, das heißt, des Bereiches, innerhalb dem Analytkonzentrationen eines festgelegten Immunoassays gemessen werden können. Für den Fall niedriger Temperaturen ist es bevorzugt, die angelegte Rechteck-Spannung fur eine gegenüber bei höheren Temperaturen verlängerte Anregungszeit aufrecht zu erhalten. Es hat sich nämlich herausgestellt, daß auch die bis zu einer Zeit von ca. 5 Sekunden nach Anlegen der Spannung gemessene Intensivität einen erheblichen Beitrag zum Signal liefert.

Eine Erhöhung der Empfindlichkeit bzw. unteren Nachweisgrenze für einen Analyten läßt sich, allein oder in Kombination mit den anderen genannten Maßnahmen erreichen, wenn die während der Durchführung der Elektrochemilumineszenz-Messung in der Meßeinheit enthaltene Reagenzlösung ein Alkalichlorid in einer Konzentration von 0,1 mmol/l bis 0,5 mol/l zugegeben ist. Bevorzugt ist dieses Alkalichlorid Natriumchlorid. Die Konzentration ist für Natriumchlorid besonders bevorzugt 0,05 mol/l bis 0,45 mol/l, besonders bevorzugt 0,35 mol/l. Darüberhinaus hat sich herausgestellt, daß die erfindungsgemäße Verwendung von Natriumchlorid zu einer Verkürzung der Meßzeit ausgenutzt werden kann. Der Stromfluß in der Zelle wird außerdem erhöht.

Überraschenderweise konnte auch eine Erhöhung des Signals erreicht werden, indem die Ausgangsspannung vor Anregung der Elektrochemilumineszenz an der Arbeitselektrode zwischen + 400 und - 400 mV, verglichen mit einer Ag/AgCl-Elektrode, betrug. Besonders bevorzugt ist ein Wert zwischen + 50 mV und - 50 mV, besonders bevorzugt 0 mV. Auch hier gelten die Werte für die Verwendung eines Platin-elektrodenpaars. Die Potentiale für andere Elektrodenmaterialien können leicht errechnet werden.

Ebenfalls eine Steigerung des Signals läßt sich erreichen durch Einstellung eines pH-Wertes zwischen 6,8 - 9,0, bevorzugt zwischen 7,0 - 8,0, besonders bevorzugt zwischen 7,25 und 7,5. Dies geschieht zweckmäßigerweise durch Einsatz eines für den Bereich geeigneten pH-Puffers.

Es hat sich gezeigt, daß das aus der WO 90/05302 bekannte und bisher ublicherweise zugesetzte Detergenz Triton X-100, das in der Praxis immer in Kombination mit dem Detergenz Tween 20 eingesetzt wurde, nicht optimal ist. Einerseits ist Triton X-100 schlecht abbaubar und damit nicht umweltverträglich. Zum anderen wurde überraschenderweise gezeigt, daß ganz bestimmte andere Detergenzien im Vergleich zu Triton X-100 zur Verbesserung das ECL-Verfahrens führen. Mit diesen speziellen Detergenzien wird eine erhöhte Signalausbeute, ein besseres Signal/Rausch-Verhältnis und damit eine erhöhte Sensitivität des Nachweisverfahrens und eine Erniedrigung der unteren Nachweisgrenze sowie eine bessere Präzision erreicht.

Als geeignet haben sich die Detergenzien ausgewählt aus der Gruppe der Fettalkoholethoxylate, worunter zum Beispiel Polidocanol (Dodecylpoly-(ethylenglycolether)ₙ), C14-E09 (Poly(ethylenglycolether)ₙ), Genapol (Isotridecylpoly(ethylenglycolether)ₙ), C8-E09 (Octylalkoholpoly(ethylenglycolether)ₙ) zu verstehen sind, Plantaren (Alkylpolyglucosid) und Octylglucosid (Octyl-beta-D-glucopyranosid) oder ein Gemisch hiervon erwiesen. Die Detergenzien werden in Konzentrationen von 0,001 bis 1,0 % eingesetzt. Die am besten geeignetste Konzentration kann für jedes Detergenz leicht ermittelt werden. Konzentrationen von 0,1 bis 0,5 % haben sich am geeignetsten erwiesen.

Zur Konservierung wurde bisher der Testzusammensetzung Natriumazid üblicherweise in einer Konzentration von 5 - 10 mM zugesetzt. Es hat sich gezeigt, daß dieses umweltschädliche Agens durch Bioban oder Oxaban ersetzt werden kann, die weitaus umweltverträglicher als Azid sind. Überraschenderweise hat sich gezeigt, daß diese Konservierungsmittel einen weiteren positiven Effekt auf das ECL-Verfahren ausüben. Im Vergleich zu Azid wird eine Erhöhung des Messignals beobachtet. Oxaban und Bioban werden dabei in Konzentrationen von 0,01 bis 1 %, bevorzugt 0,1 bis 0,5 % eingesetzt.

Die oben genannten Maßnahmen führen alleine schon zu erheblichen Verbesserungen der bekannten Verfahren. Darüber hinaus jedoch kann die Empfindlichkeit oder/und der dynamische Meßbereich von Analytnachweisen durch eine Kombination der Maßnahmen in besonders großem Umfang gesteigert werden.

Eine weitere Erniedrigung der Nachweisgrenze konnte durch Deoxigenierung erreicht werden, z. B. durch Entgasung der Reagenzlösung unter Vakuum (z. B. < 50 mbar, 2 - 4 h Raumtemperatur).

Die Steigerung der Nachweissensitivität von Analyten, beispielsweise in Immunoassays, wie nach dem Sandwich- oder kompetitiven Verfahren erlaubt weitere Vereinfachungen des Verfahrens oder der verwendeten Apparate. Beispielsweise ist es nun möglich, als Detektor eine Photodiode zu verwenden, die Systemkalibration zu vereinfachen, wegen der geringeren Meßzeit bei erhöhtem Signal die Anzahl von durchgeführten Tests in der Zeiteinheit zu erhöhen oder die Probenvolumina zu verringern.

Ebenfalls Gegenstand der Erfindung ist eine Reagenzlösung zur Messung elektrochemischer Phänomene und insbesondere zum Nachweis von Analyten, enthaltend ein elektrochemisch oxidierbares Amin, das im oxidierten Zustand ein starkes Reduktionsmittel darstellt, welche ein Alkalichlorid, bevorzugt Natriumchlorid, in einer Konzentration von 0,1 mmol/l bis 0,5 mol/l enthält und/oder deren pH-Wert zwischen 7,0 - 8,0 liegt. Zusätzlich kann noch ein Detergenz ausgewählt aus der Gruppe Fettalkoholethoxylate, Plantaren® und Octylglucosid oder ein Gemisch hiervon enthalten sein.

Die Reagenzlösung wird bevorzugt mit Bioban oder Oxaban konserviert.

Ein Apparat zur Durchführung von Nachweisen mittels Elektrochemilumineszenz ist beispielsweise in Beispiel 1 der WO 90/05302 ausführlich beschrieben. Ein erfindungsgemäßer Apparat enthält darüber hinaus ein Mittel zur Kühlung der Meßeinheit oder/und eines Flüssigkeitsbehälters auf Temperaturen zwischen 0 - 25 °C. Als Meßeinheit soll hier die Zelle verstanden werden, in der die Messung der Elektrolumineszenz vorgenommen wird. Unter Flüssigkeitsbehälter kann ein Vorratsgefäß oder aber eine Zuführung, z. B. Schlauch, für die während der Messung in der Meßeinheit befindlichen Reagenzlösung verstanden werden.

Ebenfalls Gegenstand der Erfindung ist ein Verfahren zum Nachweis eines Analyten über eine Elektrochemilumineszenzmarkierung, wobei eines der oben genannten Verfahren zur Messung elektrochemilumineszenter Phänomene verwendet wird.

Die folgenden Beispiele sollen die Erfindung näher erläutern:

### Beispiel 1

### Bestimmung von TSH mittels Elektrochemilumineszenz

Thyroid-stimulierendes Hormon (TSH) wurde über einen Sandwich-Immunoassay bestimmt. Zur Ausführung kam hierbei ein Apparat, wie er in Beispiel 1 der WO 90/05302 beschrieben ist, welcher ferner in der Meßzelle einen Permanentmagneten enthielt (Origen 1.0 von IGEN, Rockville, USA oder Magnalyser®). Dieses Instrument enthält ferner einen Photomultiplier, einen Potentiostaten, eine elektrochemische Durchflusszelle, Flüssigkeitstransfermittel und einen 50-Tube Probenrotor. Zum Nachweis wurden vereinigt:

| | | |
|---|---|---|
| Inkubationspuffer (enthaltend 6.06 g/l Tris x HCl, 1 g/l Chloracetamid, 0,1 g/l Methylisothiazolon, pH 8.0, 50 g/l Rinderserumalbumin, 10 g/l R-IgG) | | 50 µl |
| | | |
| Streptavidinüberzogene Magnetpartikel (Dynal, 2,8 µm) | 600 µg/ml | 40 µl |
| | | |
| mit DSS (Disuccinidyl Suberat) biotinylierter monoklonaler Antikörper (MAK) gegen TSH | 3,0 µg/ml | 40 µl |
| TAG: (Tris)(2,2'-Bipyridyl)Rutheniumchloridhexahydrat gebunden mit DSS an MAK gegen TSH | 1,2 µg/ml | 40 µl |
| | | |
| Probenflüssigkeit bzw. Standard | | 50 µl |
| | | |
| Resuspension (Zugabe von Reagenzlösung (BMG1) | | 100 µl |

Die streptavidinüberzogenen Magnetpartikel wurden von der Firma Deutsche Dynal GmbH, Deutschland bezogen (Dynabeads M-280 Streptavidin).

Dieses Gemisch wurde 16 Minuten bei Raumtemperatur (21°C) inkubiert und anschließend in die auf die gewünschte Meßtemperatur gebrachte Meßzelle überführt, die immobilisierten Partikel mit Reagenzlösung BMG1 gewaschen und in BMG1 vermessen.

BMG1 hat folgende Zusammensetzung:

| Reagenz | pH 7,5 g per l |
|---|---|
| KH₂PO₄*2H₂O | 27,19 |
| H₃ PO₄ | - |
| Polidocanol | 1,0 |
| Oxaban A | 1,0 |
| TPA (Tripropylamin) | 14,33 |
| KOH | 3,6 |

Die Auswirkung der Zelltemperatur auf die Signalwiederfindung und die Standardkurve für den Fall einer Rampenspannung wurde an dem Beispiel dieses TSH-Immunoassays ermittelt.

**Tabelle 1**

| Auswirkung der Zelltemperatur auf die Signalwiederfindung | | | | |
|---|---|---|---|---|
| | 21°C | 28°C | 35°C | 42°C |
| Stdabw. | 49,00 | 33,00 | 78,20 | 68,90 |
| Vk | 2,59 | 1,48 | 3,30 | 3,60 |
| UNG (2s) | 0,07 | 0,05 | 0,14 | 0,13 |
| UNG (3s) | 0,11 | 0,08 | 0,21 | 0,20 |
| b/a | 1,28 | 1,24 | 1,12 | 1,13 |
| e/a | 65,82 | 45,67 | 33,38 | 27,79 |
| Standard a | 1893 c | 2248 c | 2345 c | 1894 c |
| Standard b | 2421 c | 2779 c | 2619 c | 2139 c |
| Standard c | 6918 c | 6357 c | 5253 c | 4103 c |
| Standard d | 35660 c | 30532 c | 23695 c | 17490 c |
| Standard e | 124595 c | 102674 c | 78265 c | 52625 c |
| UNG (2s) bzw. UNG (3s): untere Nachweisgrenze definiert durch zwei 2 bzw. 3 Standardabweichungen vom Mittelwert des Standard a-Signals Stdabw.: Standardabweichung VK: Variationskoeffizient b/a: Verhältnis Meßsignal des Standards b/a e/a: Verhältnis Meßsignal des Standards e/a (dynamischer Bereich) c: counts (Meßsignal) | | | | |

Die Standards a - e hatten TSH-Konzentrationen von:
a: 0 µU/ml
b: 0,39 µU/ml
c: 3,54 µU/ml
d: 12,40 µU/ml
e: 44,30 µU/ml

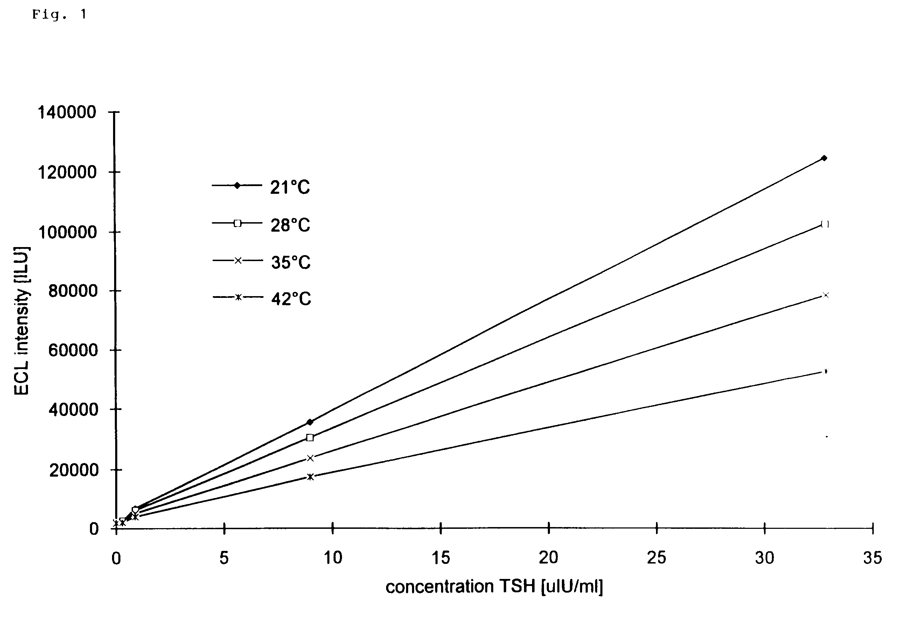

Die Auswirkung der Zelltemperatur auf die Standardkurve ist in Figur 1 gezeigt. Aufgetragen ist die detektierte Chemilumineszenz gegen die Konzentration von 5 Standards a - e mit unterschiedlicher TSH-Konzentration in µIU/ml. Es wird klar, daß die Lichtausbeute bei Rampenspannung bei niedrigen Temperaturen steigt.

### Beispiel 2

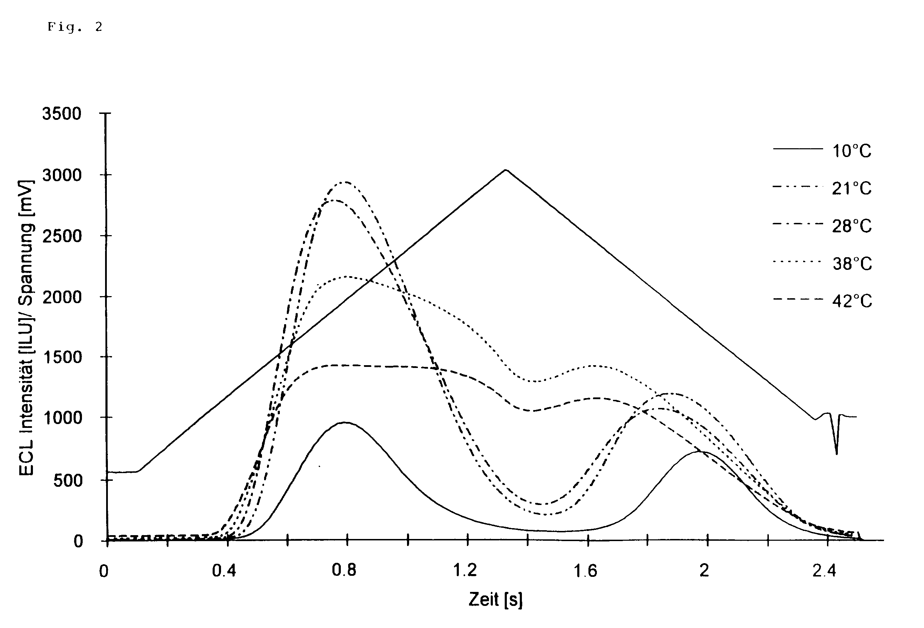

Für unterschiedliche Temperaturen zwischen 10 und 42 °C wurde der Temperatureffekt auf die Bestimmung von TSH nach Beispiel 1 in einem Puffer (BMG1) bei einer TSH-Konzentration des Standards e gemessen. Hierbei wurde der Spannungsverlauf einer sogenannten Rampenspannung benutzt, bei dem das Potential ausgehend von einer Ausgangsspannung von 565 mV über einen Zeitraum von ca. 1 Sekunde bis auf 3 V anstieg und anschließend mit dem gleichen Gefälle auf 1 V abfiel. Die Ergebnisse sind in Figur 2 dargestellt. Es wird deutlich, daß bei Verwendung einer Rampenspannung die Signalintensität von 42 nach 21 °C hin zunimmt und dann bei 10 C wieder abfällt.

### Beispiel 3:

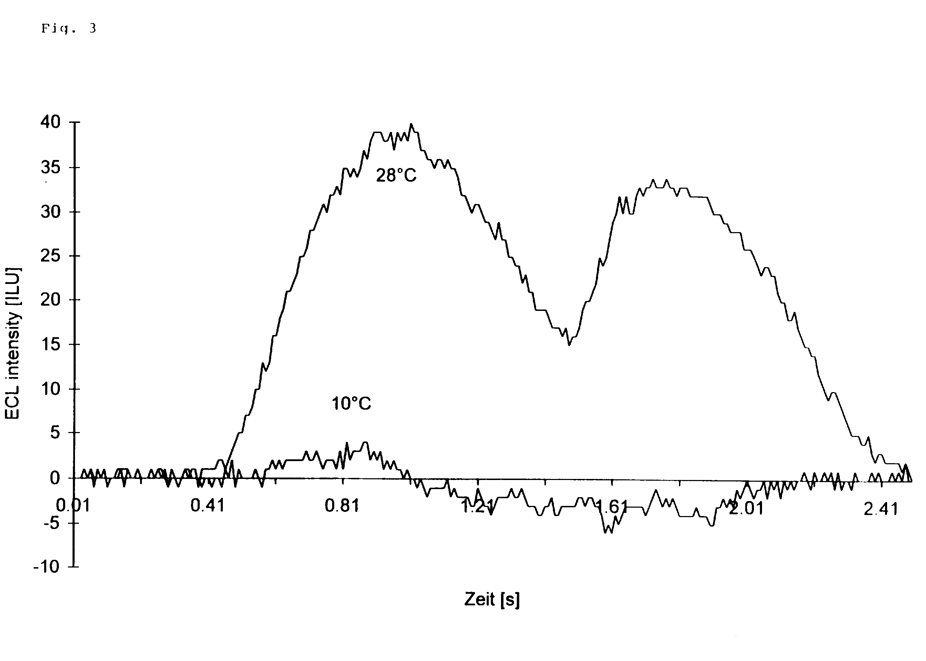

Bei den Temperaturen von 10 und 28 °C wurde der Effekt auf das Test-spezifische Hintergrundsignal, d. h. das Signal, das in Abwesenheit des biotinylierten Antikörpers allein vom TAG-Analyt-Komplex generiert wird, in dem Puffer BMG1 bestimmt. Hierbei wurde eine Rampenspannung wie in Beispiel 2 beschrieben angelegt. Die Ergebnisse sind in der Figur 3 dargestellt. Es wird deutlich, daß die Test-spezifische unspezifische Bindung des TAG-Analyt-Komplexes mit fallender Temperatur abnimmt.

### Beispiel 4

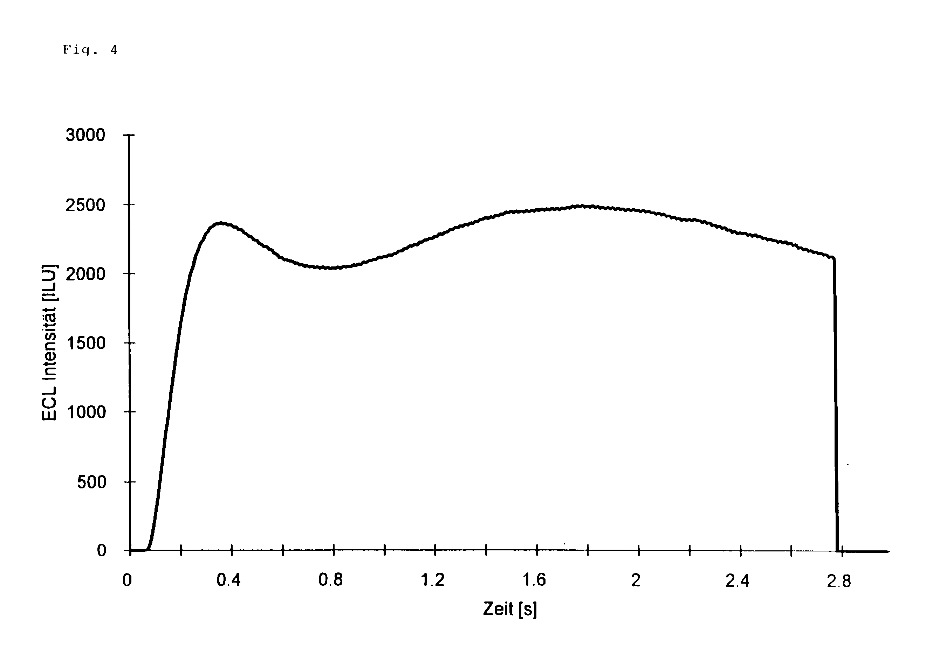

Für eine TSH-Bestimmung nach Beispiel 1 mit der Konzentration des Standards e und Puffer BMG1 wurde bei 10 °C und rechteckigem Spannungsverlauf, startend von 565 mV, der zeitliche Verlauf der Menge des detektierten Lichtes gemessen. Das Ergebnis ist in Figur 4 wiedergegeben. Es ist ersichtlich, daß ein Großteil der Strahlungsintensität bei relativ späten Zeitpunkten gemessen werden muß. Es muß angenommen werden, daß die Intensität, welche nach 0,8 Sekunden gemessen wurde, bisher entweder nicht gemessen werden konnte oder nicht gemessen wurde.

Die Nachweisgrenze bei Verwendung des rechteckigen spannungsverlaufs gegenüber Rampenspannung konnte für TSH von 0,03 µIU/ml auf 0,006 µIU/ml gesenkt werden. Der dynamische Bereich wurde von 55 auf 104 verbessert. Diese Quotienten stellen die Signalintensität des Standards mit höchster Konzentration (e) zu Signalintensität des Standards mit niedrigster Konzentration (a) dar.

### Beispiel 5

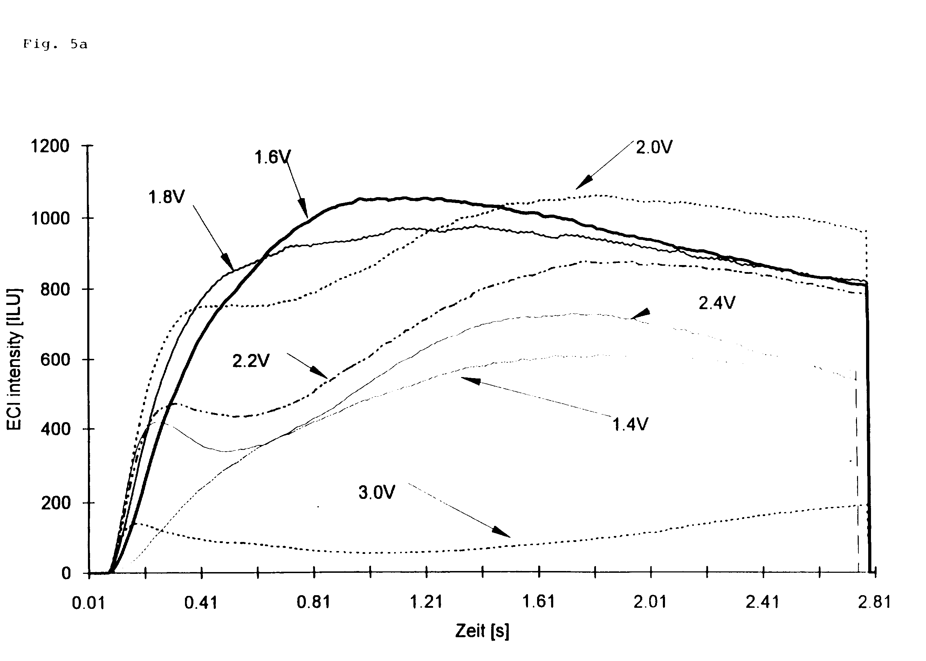

In einer Reihe von Versuchen wurde der Einfluß der Größe der für die Anregung der Elektrochemilumineszenz angelegten Endspannung auf die Signalintensität untersucht (Rechteck-spannung). Das Ergebnis ist in Figur 5a dargestellt. Spannungen zwischen 1,6 und 2,0 V lieferten gute Ergebnisse. Eine Spannung von 1,6 V erwies sich als optimal.

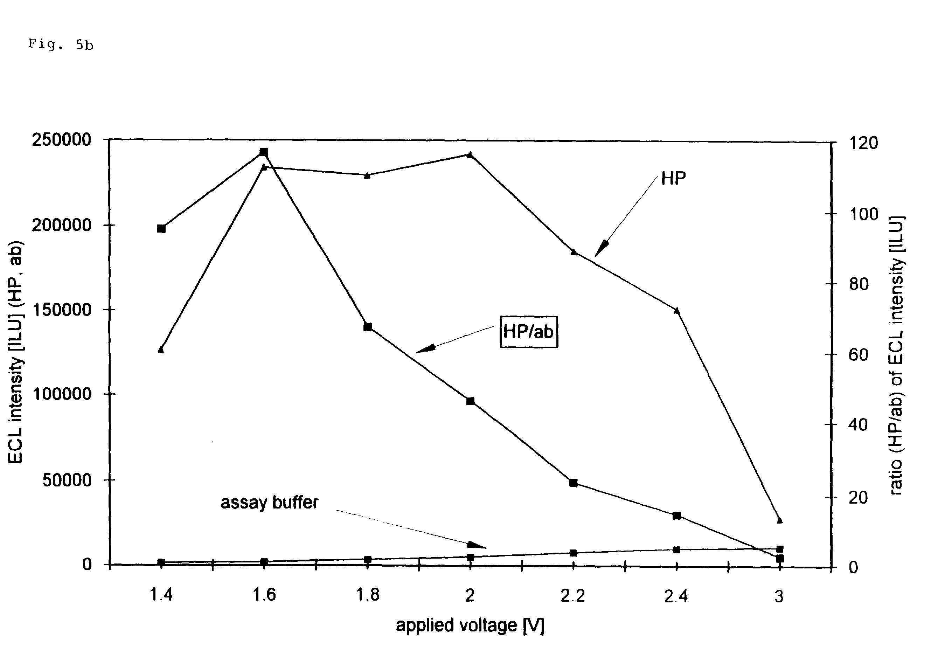

In der Figur 5b sind die Signalamplituden der Magnetpartikel (HP) dargestellt, die in der Figur 5a als Signalverläufe dargestellt wurden. Gleichzeitig wird das Background-Signal (ab) und das Verhältnis der Signalamplituden der Magnetpartikel zum Background (HP/ab) dargestellt. Wesentlich für die Wahl der zu verwendenden Spannung ist das Signal-Rausch-Verhältnis, also HP/ab. Aus der Figur 5b geht hervor, daß dieses für 1,6 V am höchsten ist.

Zur Vermessung kam eine Suspension von Magnetpartikeln, die folgendermaßen mit Elektrochemilumineszenzmarkierung beladen wurden:

Mit Streptavidin gecoatete Magnetpartikel (Dynal, 2.8 µm Durchmesser) wurden mit einem Konjugat aus einem biotinylierten polyklonalen Antikörper (IgG) gegen T4, der mit Ruthenium-bis-pyridyl-N-Hydroxy-succinimidester (IGEN, Herstellerangaben) markiert ist in einem Puffer (50 mM HEPES pH 7.4; 3% Saccharose; 2 % Rinderserumalbumin; 0.01 % Methylisothiazolon; 0,1 % Oxypyrion®) für 1 h bei 21 °C in einem Rollmixer inkubiert (20 ng Konjugat/1 mg Partikel).

Die Partikel wurden mit unmarkierten Dynal-Partikeln so verdünnt, daß sich eine Konzentration ergab, die 50.000 Einheiten auf dem ECL-Messgerat (Magnalyser, IGEN) liefert. Bis zum Gebrauch wurden die Partikel lyophilisiert. Zum Gebrauch wurden die Partikel in 4 ml Puffer (BMG1) suspendiert, um eine Konzentration von 600 µg/ml zu erreichen. Die Partikel wurden in Magnalyser Testtubes als 50 µl Suspension zusammen mit weiteren 500 µl Puffer (BMG1) überführt und vermessen.

### Beispiel 6

### Einfluß der Konzentration von Alkalihalogeniden auf die Elektrochemilumineszenz-(ECL Intensität)

In einer Versuchsreihe wurde am Beispiel des TSH-Testes nach Beispiel 1 untersucht, welchen Einfluß die Konzentration von Natriumchlorid/Kaliumchlorid auf das ECL-Signal hat. Hierbei wurde TSH-Standard e benutzt. Es wurde eine Rechteck-Spannung von 1,6 V angelegt. Ausgangsspannung war 565 mV. Der Puffer war BMG1. Die Temperatur betrug 10 °C.

Es wird klar ersichtlich, daß die Testergebnisse ohne Natriumchlorid sehr schlecht sind, während die Integrale bei den Konzentrationen 0,1 mol/l und 0,25 mol/l deutlich erhöht sind. Auch bei Kaliumchlorid ergab sich ein, wenn auch nicht so großer, Signalhub.

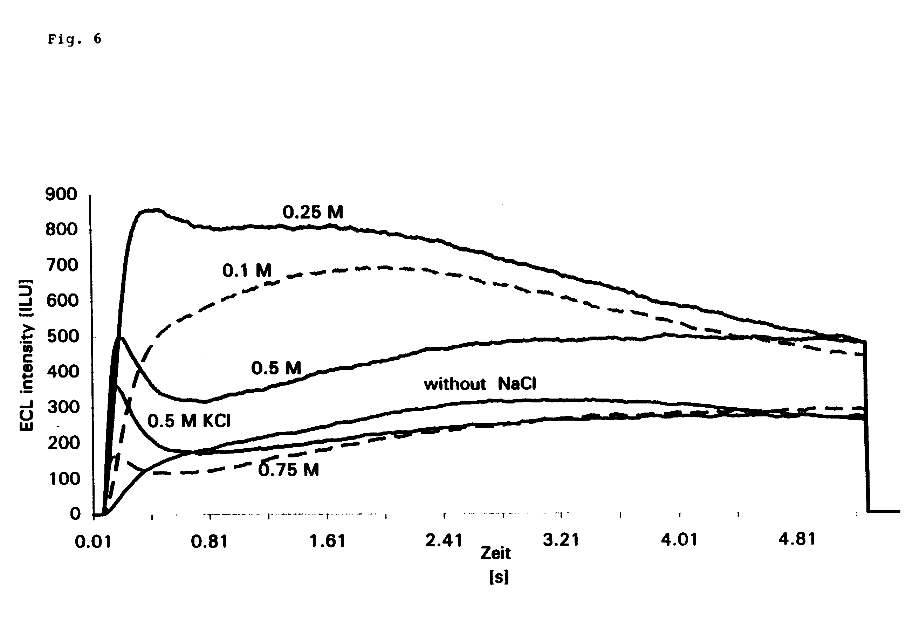

Die Ergebnisse sind in Figur 6 wiedergegeben.

### Beispiel 7

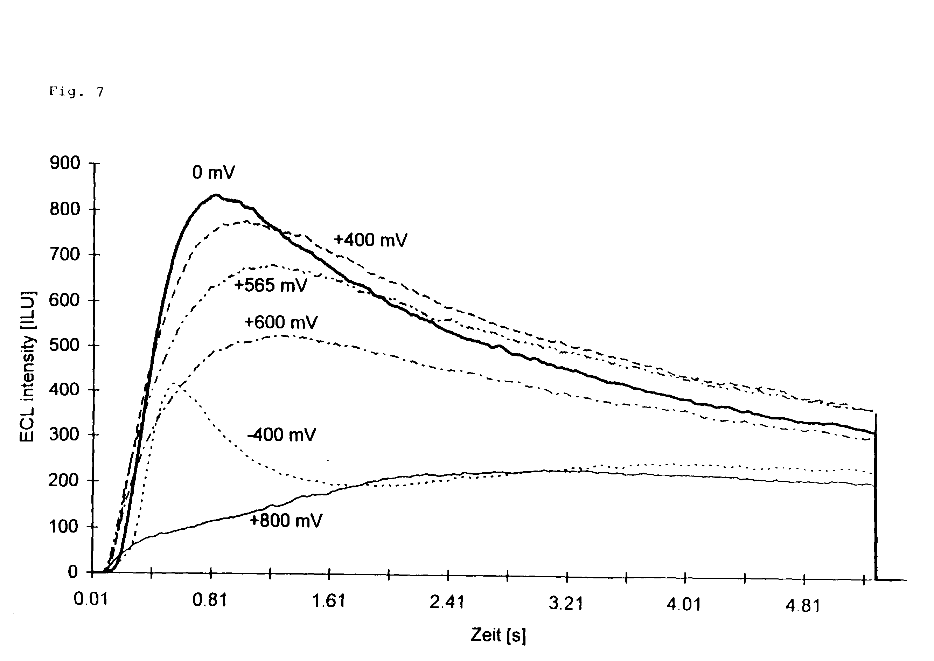

In einer Versuchsreihe wurde der Einfluß der Ausgangsspannung (Vorpolarisation der Arbeitselektrode) bei 10 °C mit Puffer BMG1 für mit TAG markierten Dynalpartikeln gemessen. Die ECL-Spannung betrug 1,6 V (Rechteck). Aus der Zeichnung in Figur 7 wird erkenntlich, daß die ECL-Intensität bei Annäherung der Ausgangsspannung an 0 mV gegen Ag/AgCl gegen ein Maximum strebt.

### Beispiel 8

In einem TSH-Test mit Standard e nach Beispiel 1 wurde der Einfluß des pHs in der Reagenzlösung gemessen. Weitere Parameter waren:

| | |
|---|---|
| Rechteck-Spannungsverlauf | 1,6 V (Maximum) |
| Ausgangsspannung | 565 mV |
| Temperatur | 10 °C |

Es wird hier klar erkenntlich, daß bei pH 7,5 die Signalintensität im gemessenen Zeitraum deutlich erhöht ist gegenüber pH 6,8, beziehungsweise den im Stand der Technik beschriebenen Puffern (Igen-AB).

Puffer BMG2 hat folgende Zusammensetzung:

| Reagenz | pH 6,8 g per l |
|---|---|
| KH₂ PO₄ *2H₂O | - |
| H₃ PO₄ | 16,4 |
| Polidocanol | 1,0 |
| Oxaban A | 1,0 |
| TPA (Tripropylamin) | 22,93 |
| KOH | 5,7 |

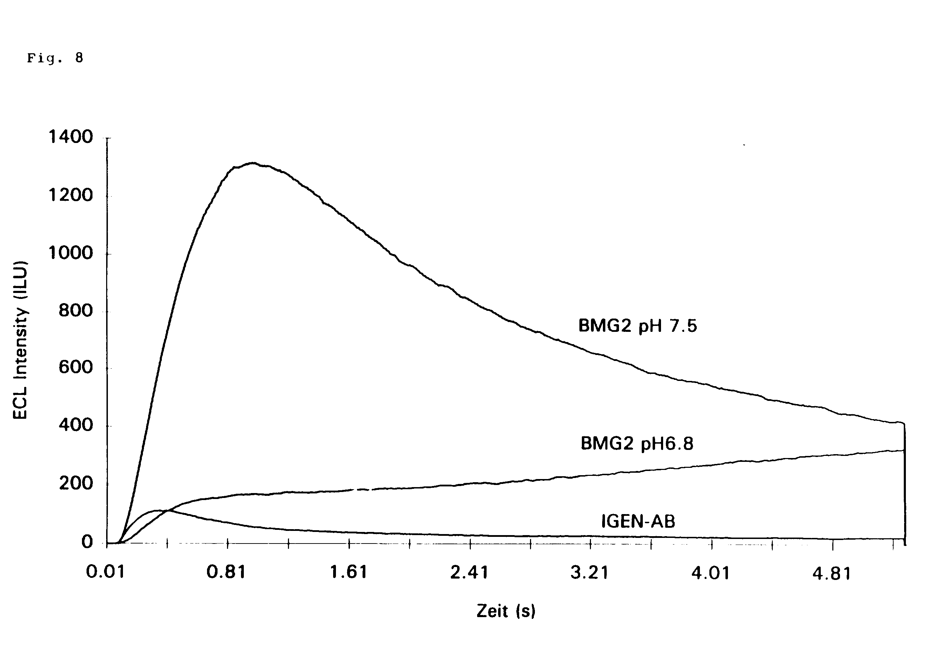

Die Ergebnisse sind in Figur 8 dargestellt. Die integrierte Intensität beträgt bei pH 6,8: 112000 und bei pH 7,5: 399000.

### Beispiel 9

Die hier durchgeführte Versuchsreihe am Beispiel eines TSH-Testes nach Beispiel 1 zeigt, daß durch Kombination der erfindungsgemäßen Maßnahmen nochmals eine erhebliche Erhöhung des ECL-Signals in einem relativ frühen Zeitraum erreicht werden kann. Die fünf abgebildeten Kurven wurden bei 10 °C und einer Elektrochemilumineszenzspannung von 1,6 V (Rechteckspannung) gemessen.

| | |
|---|---|
| Kurve 1 | BMG2, pH 6.8, Ausgangsspannung 565 mV |
| Kurve 2 | BMG1, pH 7.5, Ausgangsspannung 565 mV |
| Kurve 3 | BMG2, pH 7.5, Ausgangsspannung 565 mV |
| Kurve 4 | BMG2, pH 7.5, Ausgangsspannung 0 mV |
| Kurve 5 | BMG2, pH 7.5, 0.25 M NaCl, Ausgangsspannung 0 mV |

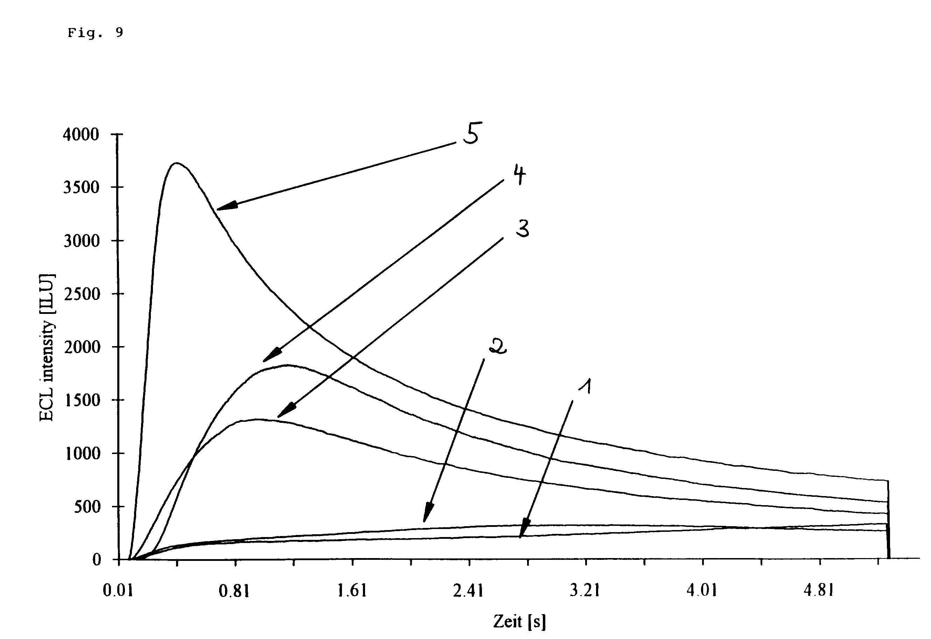

Das Ergebnis der Versuche ist in Figur 9 gezeigt.

### Beispiel 10

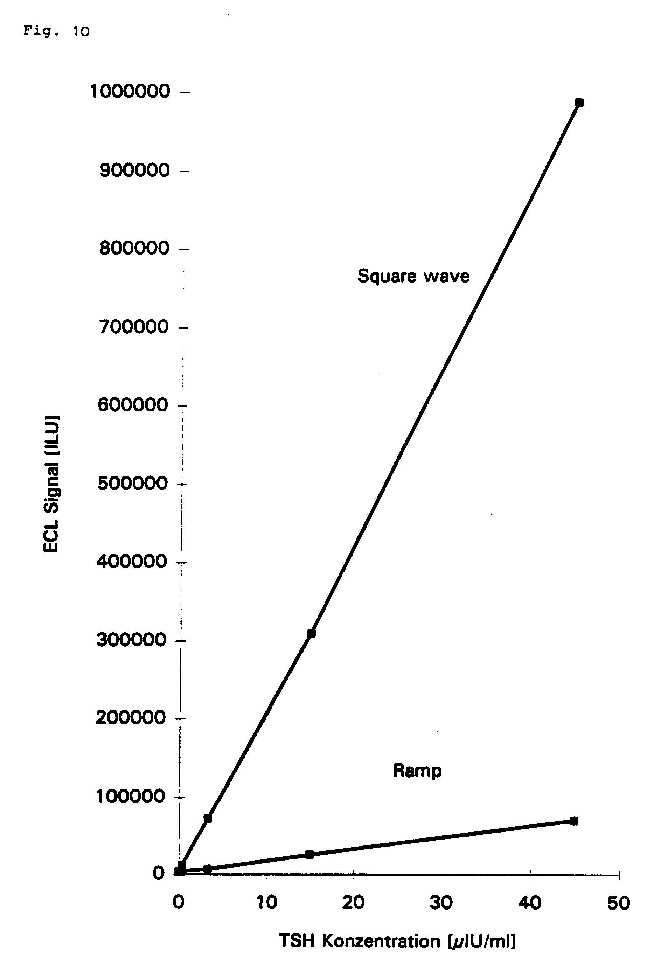

In Figur 10 sind Standardkurven für eine Bestimmung von TSH nach Beispiel 1 bei 10 °C dargestellt. Kurve 1 zeigt die Standardkurve unter Verwendung der Rampenspannung (wie Figur 2), während Kurve 2 die Standardkurve unter Verwendung einer Rechteckspannung kombiniert mit optimierten pH, Ionenstärke- und Präpolarisationsbedingungen wiedergibt. Dies zeigt, daß bei Verwendung niedriger Temperaturen die Signalintensität durch Anwendung eines rechteckigen Spannungsverlaufs noch gesteigert werden kann.

Die Nachweisgrenze von TSH konnte durch Kombination der Maßnahmen auf ca. 0,0028 µIU/ml gesenkt werden. Der dynamische Bereich wurde auf ca. 280 (Standard e/Standard a) verbessert.

### Beispiel 11

In weiteren Versuchen wurde bestätigt, daß sich der erfindungsgemäße Effekt auch für andere Analyten verifizieren läßt. In Figur 11 sind die Standardkurven für eine Bestimmung von Östradiol (E2) unter den Bedingungen des Standes Technik (Ramp) und den erfindungsgemäßen Bedingungen (10 °C, Rechteckspannung 1,6 V) verglichen. Die Empfindlichkeit (untere Nachweisgrenze) kann um den Faktor 20 (bis herunter auf ca. 1,5 pg/ml) gesteigert werden.

Fur den Nachweis von E2 wurden folgende Komponenten miteinander inkubiert:

| 1. Schritt: | |
|---|---|
| | |
| 25 µl | Puffer BMG 1 |
| 50 µl | Lösung von biotinyliertem (DSS) polyklonalem Antikörper (FAB') gegen E2 |
| 50 µl | Probe bzw. Standard (enthaltend 0.1 % Polidocanol 0.3 % Rinderserumalbumin) |
| 50 mg/ml | Dihydroxytestosteron |

Danach wurde 15 min. inkubiert.

| 2. Schritt: Zugabe von | |
|---|---|
| | |
| 25 µl | Puffer BMG 1 |
| 50 µl | Magnetpartikellösung aus Beispiel 1 |
| 50 µl | Lösung von TAG: 300 mg/ml FAB-Fragment gegen E2, markiert mit Ru(bpy)₃ ²⁺-NHS-Ester von IGEN |

Auch danach wurde 15 min. inkubiert. Dann wurden 100 µl BMG 1 zugegeben, die Suspension in den Magnalyzer überführt, die immobilisierten Magnetpartikel mit 1500 µl BMG 1 gewaschen und in BMG 1 für folgende Standards vermessen:

| | |
|---|---|
| a | 0 pg/ml |
| b | 72 pg/ml |
| c | 229 pg/ml |
| d | 529 pg/ml |
| e | 1709 pg/ml |
| f | 5032 pg/ml |

### Beispiel 12

In einer Versuchsreihe wurde der alleinige Effekt der erfindungsgemäß eingesetzten Detergenzien ermittelt. Um den Einfluß der Detergenzien auf die Signalerzeugung möglichst unabhängig von einzelnen Testparametern, d. h. zu bestimmten Analyten bestimmen zu können, wurden streptavidinüberzogene Magnetpartikel, an denen ein biotinylierter und gleichzeitig ruthenylierter Antikörper gekoppelt war, eingesetzt (HSAP: "hot-Streptavidin-Partikel").

Zum Nachweis wurden in einem Tube vereinigt:

| | |
|---|---|
| HSAP (lyophilisierte HSAP wurden in einem Tris/Polidocanol-Puffer (100 mM; 0,1 %) pH 9,0 gelöst, sodaß eine Arbeitslösung von 600 µg/ml vorlag) | 50 µl |
| | |
| PBS-Puffer (50 mM KH₂ PO₄-Puffer; 100 mM NaCl; 0,1 % RSA; pH 7,0) | 200 µl |
| | |
| Reagenzlösung (200mM KH₂ PO₄-Puffer; 100 mM TPA; pH 7,5; jeweilig getestetes Reagenz) | |

Dieses Gemisch wurde in ein Meßtube pipettiert und anschließend in die Meßzelle überführt. Die HSAP wurden mit dem Puffer AB gewaschen und in diesem Puffer die Signalausbeute vermessen.

Die verwendeten Antikörper wurden mit Biotin-DDs (Biotinylamino-3,6-dioxaoctanoyl-aminocarbonyl-heptansäure-N-hydroxysuccinimidester) biotinyliert. (Tris) (2,2'-Bipyridyl) Rutheniumchloridhexahydrat wurde mit DSS ]isuccinylsuberat) an die Antikörper gebunden.

Es wurden Dynabeads M-280 Streptavidin der Firma Deutsche Dynal GmbH, Deutschland benutzt.

Der Puffer (AB), der bei der Messung verwendet wurde, hatte folgende Zusammensetzung:

| | |
|---|---|
| KH₂ PO₄ * 2 H₂ O | 0,2 M |
| KOH | 0,076 M |
| NaCl | 0,05 mM |
| TPA (Tripropylamin) | 0,1 M |
| Detergenz | in den in der Tabelle angegebenen Konzentrationen |
| Oxaban/Bioban | 0,1/0,3 % |
| pH | 7,5 |

Als Kontrolle wurden die bisher üblichen Detergenzien Tween 20 und Triton X-100 jeweils in einer Konzentration von 0,05 % eingesetzt. Zum Vergleich wurde in der Tabelle 2 die mit diesem Detergenz erhaltene Signalausbeute als 100 % betrachtet. Als weiterer Meßwert wurde die unspezifische Signalausbeute im Puffer (AB) bestimmt und hiermit das Verhältnis der Signalausbeute HSAP/AB ermittelt. Dieses Verhältnis zwischen der Signalausbeute mit und ohne HSAP stellt ein gutes Indiz für die Sensitivität des Testes dar. Aus den Ergebnissen der Tabelle 2 ist klar zu erkennen, daß die erfindungsgemäßen Detergenzien am geeignetsten sind. Polidocanol und C8-E09 zeigen den besten Einfluß auf das Verhältnis HSAP/AB. Andere Detergenzien bewirken im Vergleich zu Tween/Triton X-100 eine Verschlechterung der Signalausbeute.

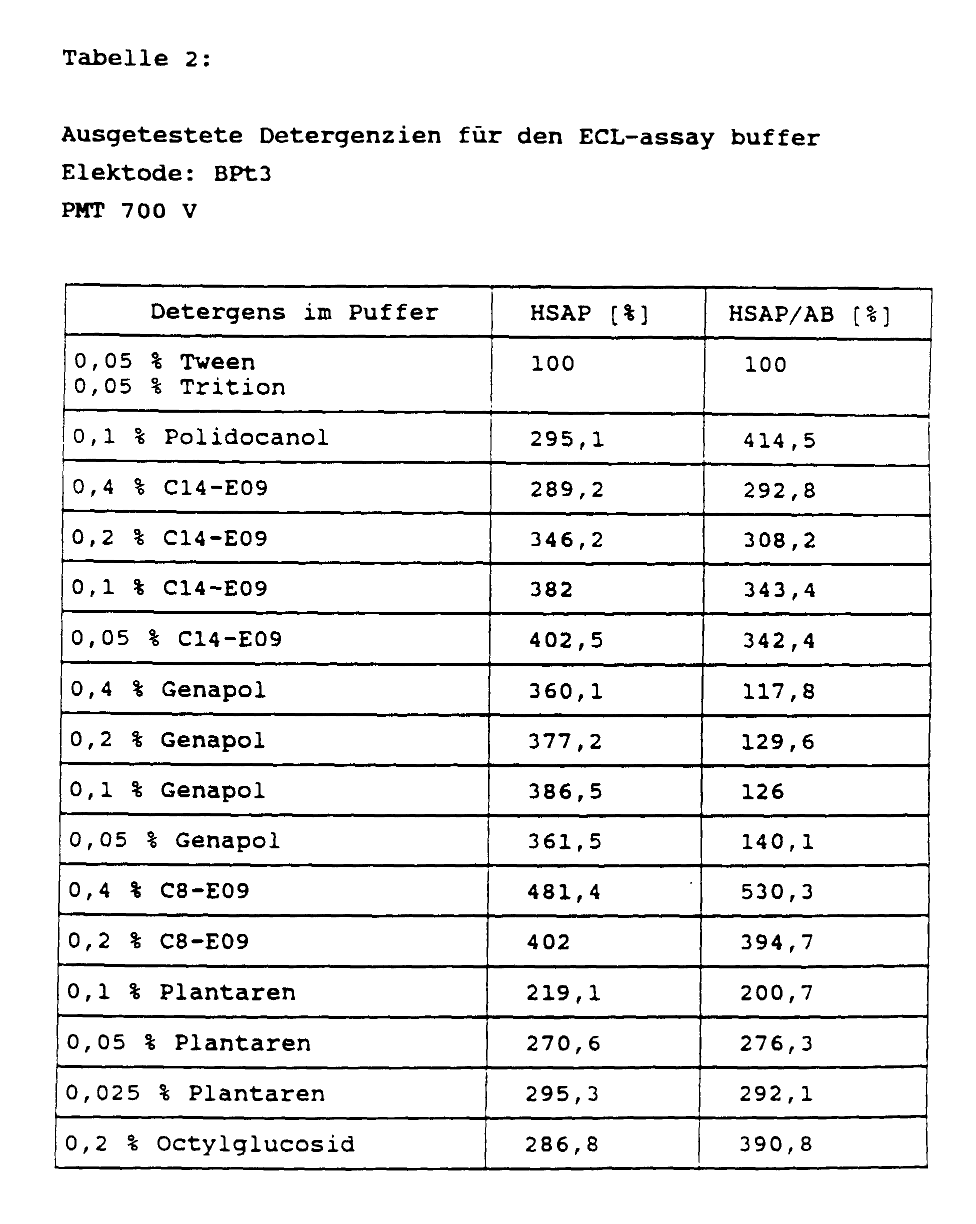

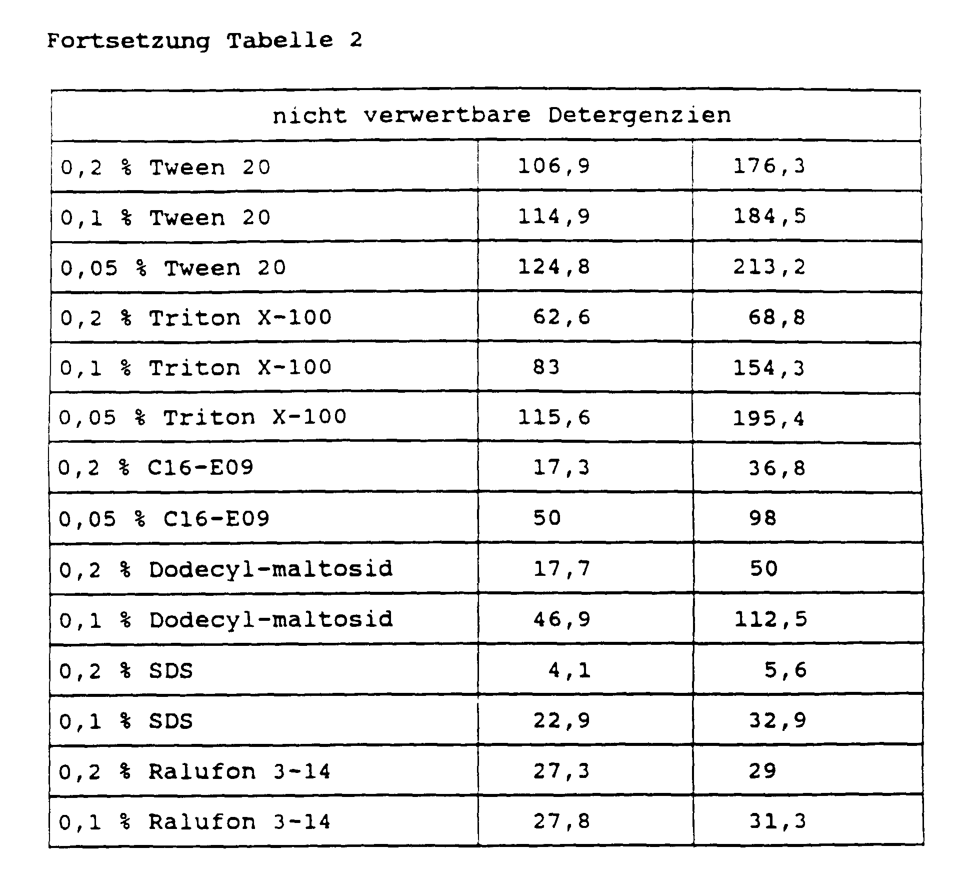

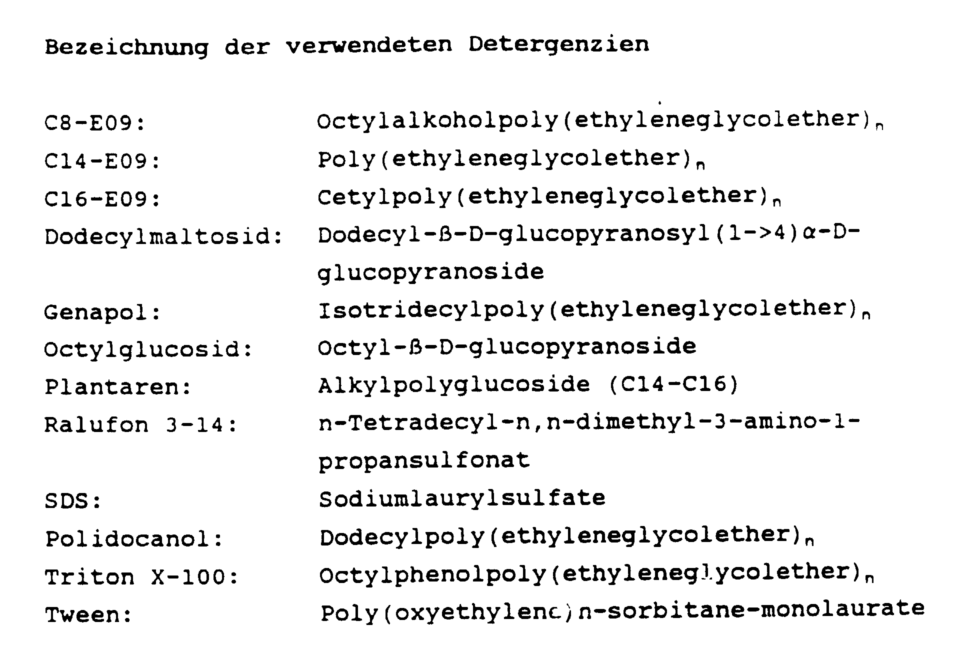

## Patentansprüche

1. Verfahren zur Messung elektrochemilumineszenter Phänomene in einer wässrigen Lösung oder aus einer an die wässrige Lösung angrenzenden festen Phase wobei die Lösung ein oxidierbares Amin und eine zur Elektrochemilumineszenz fähige Komponente enthält, Anlegen einer Spannung zur Anregung der Elektrochemilumineszenz und Messung des ausgesendeten Lichtes als Maß für die Anwesenheit der zur Elektrochemilumineszenz fähigen Komponente, dadurch gekennzeichnet, daß die Messung der Elektrochemilumineszenz bei einer Temperatur der Lösung und/oder der festen Phase durchgeführt wird, die über dem Gefrierpunkt der Lösung liegt, aber niedriger als 25 °C ist.

2. Verfahren nach Anspruch 1, dadurch gekennzeichnet, daß die Temperatur zwischen 5 °C und 20 °C liegt.

3. Verfahren nach Anspruch 1, dadurch gekennzeichnet, daß die Lösung ein Detergenz ausgewählt aus der Gruppe Fettalkoholethoxylate, Alkylpolyglucosid und Octyl-beta-D-glucopyranosid oder ein Gemisch hiervon enthält.

4. Verfahren nach Anspruch 1, dadurch gekennzeichnet, daß die Lösung ein oder mehrere Alkali- oder Erdalkalihalogenide enthält.

5. Verfahren nach Anspruch 1, dadurch gekennzeichnet, daß der pH der Lösung zwischen 6,8 und 9,0 liegt.

6. Verfahren nach Anspruch 1, dadurch gekennzeichnet, daß die Elektrochemilumineszenz durch Anlegen einer Spannung von maximal 2,0 Volt angeregt wird.

7. Verfahren nach Anspruch 1, dadurch gekennzeichnet, daß der Spannungsverlauf über die Zeit rechteckig ist.

8. Verfahren nach Anspruch 1, dadurch gekennzeichnet, daß die zur Elektrochemilumineszenz fähige Komponente eine Markierung eines Analyten, eines Analytanalogen oder einer analytspezifischen Substanz darstellt und das empfangene Licht ein Maß für die Anwesenheit des Analyten darstellt.

9. Reagenzlösung zur Messung elektrochemischer Phänomene mit einem Verfahren nach Anspruch 1 enthaltend ein elektrochemisch oxidierbares Amin, das im oxidierten Zustand ein starkes Reduktionsmittel darstellt, dadurch gekennzeichnet, daß sie zusätzlich ein Alkalichlorid in einer Konzentration von 0,1 mmol/l bis 0,5 mol/l enthält.

10. Reagenzlösung nach Anspruch 9, dadurch gekennzeichnet, daß das Alkalichlorid Natriumchlorid ist.

11. Reagenz nach Anspruch 9 oder 10, dadurch gekennzeichnet, daß das Chlorid in einer Konzentration von 0,05 mol/l bis 0,45 mol/l enthalten ist.

12. Reagenzlösung nach einem der Ansprüche 9 bis 11, dadurch gekennzeichnet, daß sie einen pH zwischen 7,0 und 8,0 aufweist.

13. Reagenzlösung nach einem der Ansprüche 9 bis 12, dadurch gekennzeichnet, daß ein Detergenz ausgewählt aus der Gruppe Fettalkoholethoxylate, Alkylpolyglucosid und Octyl-beta-D-glucopyranosid oder ein Gemisch hiervon enthalten ist.

14. Verfahren nach Anspruch 1, wobei die zur Elektrochemilumineszenz fähige Komponente an Partikel gebunden vorliegt und die Elektrochemilumineszenz durch Anlegen einer Spannung an eine Arbeitselektrode angeregt wird, dadurch gekennzeichnet, daß vor Anregung der Elektrochemilumineszenz an die Arbeitselektrode ein Potential zwischen + 400 und - 400 mV verglichen mit einer Ag/AgCl-Elektrode angelegt wird.

15. Verfahren nach Anspruch 1, wobei die zur Elektrochemilumineszenz fähige Komponente an Partikel gebunden vorliegt und die Elektrochemilumineszenz durch Anlegen einer Spannung angeregt wird, dadurch gekennzeichnet, daß die Elektrochemilumineszenz zwischen dem Redoxpotential des elektrolumineszenten Systems und + 800 mV darüber angeregt wird.

16. Apparat zur Messung elektrochemilumineszenter Phänomene, enthaltend eine Meßeinheit mit mindestens zwei Elektroden, wobei die Elektroden während der Messung mit der Reagenzlösung in Kontakt stehen, einen Detektor zur Messung des durch die Elektrochemilumineszenz erzeugten Lichtes, und einen Flüssigkeitsbehälter, dadurch gekennzeichnet, daß letzterer ein Mittel zur Kühlung der Messeinheit oder/und des Flüssigkeitsbehälters auf Temperaturen zwischen 0 und 25°C aufweist.

## Claims

1. Method for measuring electrochemiluminescent phenomena in an aqueous solution or at a solid phase contiguous to said aqueous solution, the solution containing an oxidizable amine and a component capable of generating electrochemiluminescence, wherein a voltage is applied to generate said chemiluminescence and the emitted light is a measure for the presence of the component capable of generating electrochemiluminescence. characterized in that the electrochemiluminescent effect is measured at a temperature of the solution and/or the solid phase which is above the freezing point of the solution but less than 25°C.

2. Method according to claim 1, characterized in that the temperature ranges between 5°C and 20°C.

3. Method according to claim 1, characterized in that the solution contains a detergent selected from the group consisting of fat alcohol ethoxylate, alkyl polyglucoside, octyl-beta-D-glucopyranoside or a mixture thereof.

4. Method according to claim 1. characterized in that the solution contains one or several alkali or earth alkali halogenides.

5. Method according to claim 1. characterized in that the pH of the solution ranges between 6.8 and 9.0.

6. Method according to claim 1, characterized in that the electrochemiluminescence is generated by applying a maximum voltage of 2.0 V.

7. Method according to claim 1, characterized in that a square-wave voltage is used for the measurement.

8. Method according to claim 1. characterized in that the component capable of generating electrochemiluminescence is a label of an analyte. an analyte analogon or an analyte-specific substance and the light received is a measure for the presence of the analyte.

9. Reagent solution for measuring electrochemical phenomena with the aid of a method according to claim 1 containing an electrochemically oxidizable amine which is a strong reducing agent when oxidized. characterized in that it additionally contains an alkali chloride at a concentration of 0.1 mmol/l to 0.5 mol/l.

10. Reagent according to claim 9, characterized in that the alkali chloride is sodium chloride.

11. Reagent according to claim 9 or 10, characterized in that the chloride is present in a concentration of 0.05 mol/l to 0.45 mol/l.

12. Reagent solution according to one of the claims 9 to 11 characterized in that said solution has a pH between 7.0 and 8.0.

13. Reagent solution according to claims 9 to 12. characterized in that the solution contains a detergent selected from the group consisting of fat alcohol ethoxylate. alkyl polyglucoside and octyl-beta-glucopyranoside or a mixture thereof.

14. Method according to claim 1 wherein the component capable of generating electrochemiluminescence is bound to a particle and electrochemiluminescence is generated by applying a voltage to a working electrode characterized in that before electrochemiluminescence is generated, a potential between + 400 and - 400 mV, compared to an Ag/AgCl electrode, is applied to the working electrode.

15. Method according to claim 1 wherein the component capable of generating electrochemiluminescence is bound to a particle and electrochemiluminescence is generated by applying a voltage characterized in that the electrochemiluminescence is generated between the redox potential of the electrochemiluminescent system and + 800 mV.

16. Apparatus for measuring electrochemiluminescent phenomenal comprising a measuring unit having at least two electrodes, said electrodes being in contact with the reagent solution during the measurement. a detector for measuring the light generated by electrochemiluminescence and a liquid container. characterized in that the latter has means for cooling the measuring unit and/or the liquid container to temperatures between 0 and 25°C.

## Revendications

1. Procédé pour mesurer des phénomènes électrochimioluminescents dans une solution aqueuse ou à partir d'une phase solide avoisinant la solution aqueuse, dans lequel la solution contient une amine oxydable et un constituant capable d'électrochimioluminescence, application d'une tension pour exciter l'électrochimioluminescence et mesure de la lumière émise en tant que mesure de la présence de constituants capables d'électrochimioluminescence, caractérisé en ce que la mesure d'électrochimioluminescence s'effectue à une température de la solution et/ou de la phase solide laquelle se situe au dessus du point de congélation de la solution, mais est inférieure à 25 °C.

2. Procédé selon la revendication 1, caractérisé en ce que la température se trouve entre 5 °C et 20 °C.

3. Procédé selon la revendication 1, caractérisé en ce que la solution contient un détergent choisi dans le groupe formé par les éthoxylates d'alcool gras, les polyglucosides d'alkyle et les octyl-bêta-D-glucopyranosides ou un mélange de ceux-ci.

4. Procédé selon la revendication 1, caractérisé en ce que la solution contient un ou plusieurs halogénures de métal alcalin ou alcalino-terreux.

5. Procédé selon la revendication 1, caractérisé en ce que le pH de la solution se trouve entre 6,8 et 9,0.

6. Procédé selon la revendication 1, caractérisé en ce que l'électrochimioluminescence est excitée par l'application d'une tension de 2,0 volts maximum.

7. Procédé selon la revendication 1, caractérisé en ce que la forme de la tension en fonction du temps est rectangulaire.

8. Procédé selon la revendication 1, caractérisé en ce que le constituant capable d'électrochimioluminescence représente une marque d'un analyte, d'un analogue à un analyte ou d'une substance spécifique à un analyte et que la lumière reçue représente une mesure de la présence de l'analyte.

9. Solution de réactifs pour mesurer des phénomènes électrochimiques avec un procédé selon la revendication 1, contenant une amine oxydable de manière électrochimique qui à l'état oxydé représente un agent de réduction fort caractérisé en ce qu'elle contient de plus un chlorure de métal alcalin à une concentration de 0,1 mmol/l à 0,5 mol/l.

10. Solution de réactifs selon la revendication 9, caractérisée en ce que le chlorure de métal alcalin est le chlorure de sodium.

11. Réactif selon la revendication 9 ou 10, caractérisé en ce que le chlorure est contenu à raison d'une concentration de 0,05 mol/l à 0,45 mol/l.

12. Solution de réactifs selon l'une quelconque des revendications 9 à 11, caractérisée en ce qu'elle présente un pH compris entre 7,0 et 8,0.

13. Solution de réactifs selon l'une quelconque des revendications 9 à 12, caractérisée en ce qu'un détergent choisi parmi le groupe formé par les éthoxylates d'alcool gras, les polyglucosides d'alkyle et les octyl-bêta-D-glucopyranosides ou leur mélange, est contenu.

14. Procédé selon la revendication 1, dans lequel le constituant capable d'électrochimioluminescence se trouve lié à une particule et l'électrochimioluminescence est excitée par l'application d'une tension à une électrode de travail caractérisé en ce qu'une tension comprise entre +400 et -400 mV, compensée par une électrode Ag/AgCl, est appliquée à l'électrode de travail avant excitation de l 'électrochimioluminescence.

15. Procédé selon la revendication 1, dans lequel le constituant capable d'électrochimioluminescence se trouve lié à une particule et l'électrochimioluminescence est excitée par l'application d'une tension, caractérisé en ce que l'électrochimioluminescence est excitée de plus entre le potentiel rédox du système électroluminescent et +800 mV.

16. Appareil pour mesurer des phénomènes d'électrochimioluminescence comprenant une unité de mesure avec au moins deux électrodes, dans lequel les électrodes restent en contact avec la solution de réactifs pendant la mesure, un détecteur pour mesurer la lumière produite par l'électrochimioluminescence et un récipient pour liquide, caractérisé en ce que ce dernier présente un moyen pour refroidir l'unité de mesure ou/et le récipient pour liquide à des températures comprises entre 0 et 25 °C.
